(19) Europäisches Patentamt
European Patent Office
Office européen des brevets

(11) EP 2 136 622 B1

(12) **EUROPEAN PATENT SPECIFICATION**

(45) Date of publication and mention of the grant of the patent:
**08.08.2012 Bulletin 2012/32**

(21) Application number: **07752593.9**

(22) Date of filing: **08.03.2007**

(51) Int Cl.:
***A01N 25/30*** *(2006.01)*

(86) International application number:
**PCT/US2007/005905**

(87) International publication number:
**WO 2008/111928 (18.09.2008 Gazette 2008/38)**

(54) **HYDROLYSIS RESISTANT ORGANOMODIFIED TRISILOXANE SURFACTANTS**

HYDROLYSERESISTENTE ORGANISCH MODIFIZIERTE TRISILOXAN-TENSIDE

AGENTS TENSIOACTIFS DE TRISILOXANE MODIFIÉS ORGANIQUEMENT RÉSISTANT À UNE HYDROLYSE

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**30.12.2009 Bulletin 2009/53**

(60) Divisional application:
**10005536.7 / 2 236 028**
**10005538.3 / 2 236 029**

(73) Proprietor: **Momentive Performance Materials Inc.**
**Wilton, CT 06897 (US)**

(72) Inventors:
- **POLICELLO, George, A.**
  **Ossining, NY 10562 (US)**
- **LEATHERMAN, Mark, D.**
  **Stamford, CT 06907 (US)**
- **PENG, Wenqing**
  **Shanghai 201203 (CN)**
- **RAJARAMAN, Suresh, K.**
  **Newburg, NY 12550 (US)**
- **XIA, Sophia**
  **Pudong, Shanghai 200135 (CN)**

(74) Representative: **Thoma, Michael et al**
**Lorenz-Seidler-Gossel**
**Rechtsanwälte-Patentanwälte**
**Widenmayerstrasse 23**
**80538 München (DE)**

(56) References cited:
**EP-A- 1 053 678** **WO-A-94/22311**
**WO-A-2005/013693**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 136 622 B1

## Description

FIELD OF THE INVENTION

The present invention relates to trisiloxane surfactant compositions that exhibit resistance to hydrolysis over a wide pH range. More particularly the present invention relates to such hydrolysis resistant trisiloxane surfactants having a resistance to hydrolysis between a pH of about 3 to a pH of about 12.

BACKGROUND OF THE INVENTION

The topical application of liquid compositions to the surfaces of both animate and inanimate objects to effect a desired change involve the processes of controlling wetting, spreading, foaming, detergency, and the like. When used in aqueous solutions to improve the delivery of active ingredients to the surface being treated, trisiloxane type compounds have been found to be useful in enabling the control of these processes to achieve the desired effect. However, the trisiloxane compounds may only be used in a narrow pH range, ranging from a slightly acidic pH of 6 to a very mildly basic pH of 7.5. Outside this narrow pH range, the trisiloxane compounds are not stable to hydrolysis undergoing a rapid decomposition.
Polyalkylene modified trisiloxanes are disclosed by each of documents EP-A-1053678, WO 94/22311 and WO 2005-013693.

SUMMARY OF THE INVENTION

The present invention provides for an agricultural composition comprising a silicone composition comprising a trisiloxane compound or compositions thereof having the formula I, and further comprising an agriculturally active component, said agricultural composition having an enhanced resistance to hydrolysis, as defined by claim 1.
Trisiloxane compound I has the formula:

$$M^1 D^1 M^2$$

wherein

$$M^1 = (R^1)(R^2)(R^3)SiO_{1/2}$$

$$M^2 = (R^4)(R^5)(R^6)SiO_{1/2}$$

$$D^1 = (R^7)(Z)SiO_{2/2}$$

where
$R^1$ is selected from a branched or linear hydrocarbon group consisting of 2 to 4 carbons, aryl, and an alkyl hydrocarbon group of 4 to 9 carbons containing aryl substituents of 6 to 20 carbon atoms; $R^2$, $R^3$ $R^4$, $R^5$, $R^6$ and $R^7$ are each independently selected from the group consisting of 1 to 4 carbon monovalent hydrocarbon radicals, aryl, and a hydrocarbon group of 4 to 9 carbons containing an aryl group; Z is an alkyleneoxide group of the general formula: $R^8(C_2H_4O)_a(C_3H_6O)_b(C_4H_8O)_cR^9$, where $R^8$ is a linear or branched divalent hydrocarbon radical of 2, 3, 5, 6, 7, 8, or 9 carbon atoms; $R^9$ is selected from the group consisting of H, monovalent hydrocarbon radicals of from 1 to 6 carbon atoms and acetyl the subscripts a, b and c are zero or positive and satisfy the following relationships:

$$2 \leq a + b + c \leq 20 \text{ with } a \geq 2.$$

Preferred embodiments of the invention are defined in the further dependent claims.
When the subscript a satisfies the condition $2 \leq a \leq 5$ it is advisable to utilize a co-surfactant as hereinafter set forth in order to obtain the benefit of the compositions of the present invention.

DETAILED DESCRIPTION OF THE INVENTION

As used herein, integer values of stoichiometric subscripts refer to molecular species and non-integer values of stoichiometric subscripts refer to a mixture of molecular species on a molecular weight average basis, a number

average basis or a mole fraction basis. In the case of mixtures of the compounds of the present invention, it should be readily apparent that the stoichiometric subscripts of mixtures will have average values for the subscripts that may be either integral or non-integral in contrast to those of pure compounds.

The present invention provides for an agricultural composition comprising a silicone composition comprising a trisiloxane compound or compositions thereof useful as a surfactant having the formula I

Trisiloxane compound I has the formula:

$$M^1 D^1 M^2$$

wherein

$M^1 = (R^1)(R^2)(R^3)SiO_{1/2}$

$M^2 = (R^4)(R^5)(R^6)\ SiO_{1/2}$

$D^1 = (R^7)(Z)\ SiO_{2/2}$

where

$R^1$ is selected from a branched or linear hydrocarbon group consisting of 2 to 4 carbons, aryl, and an alkyl hydrocarbon group of 4 to 9 carbons containing an aryl substituents of 6 to 20 carbon atoms; $R^2$, $R^3$ $R^4$, $R^5$ $R^6$ and $R^7$ are each independently selected from the group consisting of 1 to 4 carbon monovalent hydrocarbon radicals, aryl, and a hydrocarbon group of 4 to 9 carbons containing an aryl group. Z is an alkyleneoxide group of the general formula: $R^8(C_2H_4O)_a(C_3H_6O)_b(C_4H_8O)_cR^9$ , where $R^8$ is a linear or branched divalent hydrocarbon radical of 2, 3, 5, 6, 7, 8, or 9 carbon atoms; $R^9$ is selected from the group consisting of H, monovalent hydrocarbon radicals of from 1 to 6 carbon atoms and acetyl the subscripts a, b and c are zero or positive and satisfy the following relationships:

$$2 \leq a + b + c \leq 20 \text{ with } a \geq 2.$$

When the subscript a satisfies the condition $2 \leq a \leq 5$ it is advisable to utilize a co-surfactant as hereinafter set forth in order to obtain the benefit of the compositions of the present invention.

One method of producing the composition of the present invention is to react a molecule of the following formula:

MDHM

where $D^H$ is the hydride precursor to the D' structural unit in the composition of the present invention, wherein the definitions and relationships are later defined and consistent with those defined above, under hydrosilylation conditions, with an olefinically modified polyalkyleneoxide, such as allyloxypolyethyleneglycol, or methallyloxypolyalkyleneoxide, which are incorporated here as examples, and not set forth to limit other possible olefinically modified alkyleneoxide components. As use herein the phrase "olefinically modified polyalkyleneoxide" is defined as a molecule possessing one or more alkyleneoxide groups containing one or more, terminal or pendant, carbon-carbon double bonds. The polyether (the precursor to the substituents Z, Z' or Z") is an olefinically modified polyalkyleneoxide (hereinafter referred to as "polyether") is described by the general formula :

$$CH_2= CH(R^{28})(R^{29})_jO(R^{30})_k(C_2H_4O)_m(C_3H_6O)_n(C_4H_8O)_pR^{31}$$

where

$R^{28}$ is H or methyl; $R^{29}$ is a divalent alkyl radical of 1 to 6 carbons where the subscript j may be 0 or 1; $R^{30}$ is $-C_2H_4O-$, where the subscript k may be 0 or 1; $R^{31}$ is H, a monofunctional hydrocarbon radical of 1 to 6 carbons, or acetyl and the subscripts m, n and p are zero or positive and satisfy the relationship $2 \leq m + n + p \leq 20$ with $m \geq 2$. When the polyether is composed of mixed oxyalkyleneoxide groups (i.e. oxyethylene, oxypropylene and oxybutylene) the units may be blocked, or randomly distributed. One skilled in the art will understand the advantages of using a blocked or random configuration. Illustrative examples of blocked configurations are: - $(\text{oxyethylene})_a(\text{oxypropylene})_b$-; -$(\text{oxybutylene})_c(\text{oxyethylene})_a$-; and -$(\text{oxypropylene})_b(\text{oxyethylene})_a(\text{oxybutylene})_c$-.

Illustrative examples of the polyether are provided below, but not limited to:

$CH_2=CHCH_2O(CH_2CH_2O)_8H$; $CH_2=CHCH_2O(CH_2CH_2O)_8CH_3$;

$CH_2=CHCH_2O(CH_2CH_2O)_4(CH_2CH(CH_3)O)_5H$;
$CH_2=CHO(CH_2CH_2O)_5(CH_2CH(CH_3)O)_5H$;
$CH_2=C(CH_3)CH_2O(CH_2CH_2O)_4(CH_2CH(CH_3)O)_5C(=O)CH_3$;
$CH_2=CHCH_2O(CH_2CH_2O)_5(CH_2CH(CH_3)O)_2(CH_2CH(CH_2CH_3)O)_2H$

Polyether modified siloxanes are prepared in the normal manner through the use of a hydrosilylation reaction to graft the olefinically modified (i.e. vinyl, allyl or methallyl) polyalkyleneoxide onto the hydride (SiH) intermediate of the trisiloxane of the present invention.

A preferred embodiment of trisiloxane compound formula I is where $R^1$ and $R^4$ are selected from a branched or linear hydrocarbon group consisting of 2 to 4 carbons, aryl, an alkyl hydrocarbon group of 4 to 9 carbons containing an aryl substituents of 6 to 20 carbon atoms; More preferably 3 to 4 carbons or aryl. $R^2$, $R^3$, $R^5$ $R^6$ and $R^7$ are each independently selected from the group consisting of 1 to 4 carbon monovalent hydrocarbon radicals, aryl, and a hydrocarbon group of 4 to 9 carbons containing an aryl group; more preferably 1 to 2 carbon monohydrocarbon radicals and aryl; most preferably methyl. Z is an alkyleneoxide group of the general formula:

$R^8(C_2H_4O)_a(C_3H_6O)_b(C_4H_8O)_cR^9$, where $R^8$ is a linear or branched divalent hydrocarbon radical of 2, 3, 5, 6, 7, 8, or 9 carbon atoms, more preferably 3 to 7 carbons; most preferably 3 to 6 carbons. Subscripts a, b and c are zero or positive and satisfy the following relationships:

$2 \leq a + b + c \leq 20$ with $a \geq 2$; preferably a is 5 to 20, more preferably 5 to 8; preferably b is 0 to 10; more preferably 0 to 5; preferably c is 0 to 8, more preferably 0 to 4. $R^9$ is selected from the group consisting of H, monovalent hydrocarbon radicals of from 1 to 6 carbon atoms and acetyl.

Another preferred embodiment of trisiloxane compound formula I is where $R^1$, $R^4$ and $R^7$ are selected from a branched or linear hydrocarbon group consisting of 2 to 4 carbons, and aryl; more preferably 3 to 4 carbons. $R^2$, $R^3$, $R^5$ and $R^6$ are each independently selected from the group consisting of 1 to 4 carbon monovalent hydrocarbon radicals, and aryl; more preferably 1 to 2 carbons; most preferably methyl. Z is as described above.

Precious metal catalysts suitable for making polyether substituted siloxanes are also well known in the art and comprise complexes of rhodium, ruthenium, palladium, osmium, iridium, and /or platinum. Many types of platinum catalysts for this SiH olefin addition reaction are known and such platinum catalysts may be used to generate the compositions of the present invention. The platinum compound can be selected from those having the formula ($PtCl_2$Olefin) and H ($PtCl_3$Olefin) as described in U.S. Pat. No. 3,159,601. A further platinum containing material can be a complex of chloroplatinic acid with up to 2 moles per gram of platinum of a member selected from the class consisting of alcohols, ethers, aldehydes and mixtures thereof as described in U.S. Pat. No.3,22 0,972. Yet another group of platinum containing materials useful in this present invention is described in U.S. Pat. Nos. 3,715,334; 3,775,452 and 3,814,730 (Karstedt). Additional background concerning the art may be found in J.L. Spier, "Homogeneous Catalysis of Hydrosilation by Transition Metals", in Advances in Organometallic Chemistry, volume 17, pages 407 through 447, F.G.A. Stone and R. West editors, published by Academic Press (New York, 1979). Those skilled in the art can easily determine an effective amount of platinum catalyst. Generally an effective amount ranges from about 0.1 to 50 parts per million of the total organomodified trisiloxane composition.

The compositions of the present invention exhibit an enhanced resistance to hydrolysis outside a pH range ranging from 6 to 7.5, herewith defined as an extreme environment. Enhanced resistance to hydrolysis can be demonstrated by a variety of tests but as used herein enhanced resistance to hydrolysis means 50 mole percent or more of the hydrolysis resistant composition of the present invention remains unchanged or unreacted after a period of a twenty-four exposure to aqueous acidic conditions where the solution has a pH lower than 6 or after a period of a twenty-four hour exposure to aqueous basic conditions where the solution has a pH greater than 7.5. Under acidic conditions the compositions of the present invention show a survival of 50 mole percent of the original concentration or greater at a pH of 5 or less for a period of time in excess of 48 hours; specifically the compositions of the present invention show a survival of 50 mole percent or greater at a pH of 5 or less for a period of time in excess of 2 weeks; more specifically the compositions of the present invention show a survival of 50 mole percent or greater at a pH of 5 or less for a period of time in excess of 1 month; and most specifically the compositions of the present invention show a survival of 50 mole percent or greater at a pH of 5 or less for a period of time in excess of 6 months. Under basic conditions the compositions of the present invention show a survival of 50 mole percent or greater at a pH of 8 or more for a period of time in excess of 2 weeks; specifically the compositions of the present invention show a survival of 50 mole percent or greater at a pH of 8 or more for a period of time in excess of 4 weeks; more specifically the compositions of the present invention show a survival of 50 mole percent or greater at a pH of 8 or more for a period of time in excess of 6 months; and most specifically the compositions of the present invention show a survival of 50 mole percent or greater at a pH of 8 or more for a period of time in excess of 1 year.

Uses for the compositions of the present intention:

A. Pesticide - Agriculture, Horticulture, Turf; Ornamental and Forestry:

Many pesticide applications require the addition of an adjuvant to the spray mixture to provide wetting and spreading on foliar surfaces. Often that adjuvant is a surfactant, which can perform a variety of functions, such as increasing spray droplet retention on difficult to wet leaf surfaces, enhance spreading to improve spray coverage, or to provide penetration of the herbicide into the plant cuticle. These adjuvants are provided either as a tank-side additive or used as a component in pesticide formulations.

Typical uses for pesticides include agricultural, horticultural, turf, ornamental, home and garden, veterinary and forestry applications.

The pesticidal compositions of the present invention also include at least one pesticide, where the organomodified trisiloxane surfactant of the present invention is present at an amount sufficient to deliver between 0.005% and 2% to the final use concentration, either as a concentrate or diluted in a tank mix. Optionally the pesticidal composition may include excipients, co-surfactants, solvents, foam control agents, deposition aids, drift retardants, biologicals, micronutrients, fertilizers and the like. The term pesticide means any compound used to destroy pests, e.g., rodenticides, insecticides, miticides, fungicides, and herbicides. Illustrative examples of pesticides which can be employed include, but are not limited to, growth regulators, photosynthesis inhibitors, pigment inhibitors, mitotic disrupters, lipid biosynthesis inhibitors, cell wall inhibitors, and cell membrane disrupters. The amount of pesticide employed in compositions of the invention varies with the type of pesticide employed. More specific examples of pesticide compounds that can be used with the compositions of the invention are, but not limited to, herbicides and growth regulators, such as: phenoxy acetic acids, phenoxy propionic acids, phenoxy butyric acids, benzoic acids, triazines and s-triazines, substituted ureas, uracils, bentazon, desmedipham, methazole, phenmedipham, pyridate, amitrole, clomazone, fluridone, norflurazone, dinitroanilines, isopropalin, oryzalin, pendimethalin, prodiamine, trifluralin, glyphosate, glufosinate, sulfonylureas, imidazolinones, pyridinecarboxylic acids, clethodim, diclofop-methyl, fenoxaprop-ethyl, fluazifop-p-butyl, haloxyfop-methyl, quizalofop, sethoxydim, dichlobenil, isoxaben, and bipyridylium compounds.

Fungicide compositions that can be used with the present invention include, but are not limited to, aldimorph, tridemorph, dodemorph, dimethomorph; flusilazol, azaconazole, cyproconazole, epoxiconazole, furconazole, propiconazole, tebuconazole and the like; imazalil, thiophanate, benomyl carbendazim, chlorothialonil, dicloran, trifloxystrobin, fluoxystrobin, dimoxystrobin, azoxystrobin, furcaranil, prochloraz, flusulfamide, famoxadone, captan, maneb, mancozeb, dodicin, dodine, and metalaxyl.

Insecticide, larvacide, miticide and ovacide compounds that can be used with the composition of the present invention, but not limited to, Bacillus thuringiensis, spinosad, abamectin, doramectin, lepimectin, pyrethrins, carbaryl, primicarb, aldicarb, methomyl, amitraz, boric acid, chlordimeform, novaluron, bistrifluron, triflumuron, diflubenzuron, imidacloprid, diazinon, acephate, endosulfan, kelevan, dimethoate, azinphos-ethyl, azinphos-methyl, izoxathion, chlorpyrifos, clofentezine, lambda-cyhalothrin, permethrin, bifenthrin, cypermethrin and the like.

The pesticide may be a liquid or a solid. If a solid, it is preferable that it is soluble in a solvent, or the organomodified trisiloxanes of the present invention, prior to application, and the silicone may act as a solvent, or surfactant for such solubility or additional surfactants may perform this function.

Agricultural Excipients:

Buffers, preservatives and other standard excipients known in the art also may be included in the composition.

Solvents may also be included in compositions of the present invention. These solvents are in a liquid state at room temperature. Examples include water, alcohols, aromatic solvents, oils (i.e. mineral oil, vegetable oil, silicone oil, and so forth), lower alkyl esters of vegetable oils, fatty acids, ketones, glycols, polyethylene glycols, diols, paraffinics, and so forth. Particular solvents would be 2, 2, 4-trimethyl, 1-3-pentane diol and alkoxylated (especially ethoxylated) versions thereof as illustrated in US Patent No. 5,674,832 herein incorporated by reference, or n-methyl-pyrrilidone.

Co-surfactants:

Moreover, other co-surfactants, which have short chain hydrophobes that do not interfere with superspreading as described in US Patents Nos. 5,558,806; 5,104,647; and 6,221,811 are herein included by reference.

The co-surfactants useful herein include nonionic, cationic, anionic, amphoteric, zwitterionic, polymeric surfactants, or any mixture thereof. Surfactants are typically hydrocarbon based, silicone based or fluorocarbon based.

Useful surfactants include alkoxylates, especially ethoxylates, containing block copolymers including copolymers of ethylene oxide, propylene oxide, butylene oxide, and mixtures thereof; alkylarylalkoxylates, especially ethoxylates or propoxylates and their derivatives including alkyl phenol ethoxylate; arylarylalkoxylates, especially ethoxylates or

propoxylates. and their derivatives; amine alkoxylates, especially amine ethoxylates; fatty acid alkoxylates; fatty alcohol alkoxylates; alkyl sulfonates; alkyl benzene and alkyl naphthalene sulfonates; sulfated fatty alcohols, amines or acid amides; acid esters of sodium isethionate; esters of sodium sulfosuccinate; sulfated or sulfonated fatty acid esters; petroleum sulfonates; N-acyl sarcosinates; alkyl polyglycosides; alkyl ethoxylated amines; and so forth.

Specific examples include alkyl acetylenic diols (SURFONYL-Air Products), pyrrilodone based surfactants (e.g., SURFADONE - LP 100 - ISP), 2-ethyl hexyl sulfate, isodecyl alcohol ethoxylates (e.g., RHODASURF DA 530 - Rhodia), ethylene diamine alkoxylates (TETRONICS - BASF), and ethylene oxide/propylene oxide copolymers (PLURONICS - BASF) and Gemini type surfactants (Rhodia).

Preferred surfactants include ethylene oxide/propylene oxide copolymers (EO/PO); amine ethoxylates; alkyl polyglycosides; oxo-tridecyl alcohol ethoxylates, and so forth.

In a preferred embodiment, the agrochemical composition of the present invention further comprises one or more agrochemical ingredients. Suitable agrochemical ingredients include, but not limited to, herbicides, insecticides, growth regulators, fungicides, miticides, acaricides, fertilizers, biologicals, plant nutritionals, micronutrients, biocides, paraffinic mineral oil, methylated seed oils (i.e. methylsoyate or methylcanolate), vegetable oils (such as soybean oil and canola oil), water conditioning agents such as Choice® (Loveland Industries, Greeley, CO) and Quest (Helena Chemical, Collierville, TN), modified clays such as Surround® (Englehard Corp.), foam control agents, surfactants, wetting agents, dispersants, emulsifiers, deposition aids, antidrift components, and water.

Suitable agrochemical compositions are made by combining, in a manner known in the art, such as, by mixing one or more of the above components with the organomodified trisiloxane of the present invention, either as a tank-mix, or as an "In-can" formulation. The term "tank-mix" means the addition of at least one agrochemical to a spray medium, such as water or oil, art the point of use. The term "In-can" refers to a formulation or concentrate containing at least one agrochemical component. The "In-can" formulation may then diluted to use concentration at the point of use, typically in a Tank-mix, or it may be used undiluted.

B. Coatings

Typically coatings formulations will require a wetting agent or surfactant for the purpose of emulsification, compatibilization of components, leveling, flow and reduction of surface defects. Additionally, these additives may provide improvements in the cured or dry film, such as improved abrasion resistance, antiblocking, hydrophilic, and hydrophobic properties. Coatings formulations may exists as, Solvent-borne coatings, water-borne coatings and powder coatings.

The coatings components may be employed as: Architecture coatings; OEM product coatings such as Automotive coatings and coil coatings; Special Purpose coatings such as industrial maintenance coatings and marine coatings;

Typical resin types include: Polyesters, alkyds, acrylics, polyurethans and epoxies.

Experimental

The hydride intermediates for the organomodified trisiloxane surfactant compositions of the present invention, as well as comparative compositions were prepared as described in the following examples.

PREPARATION EXAMPLE 1

1,5-di(t-butyl)-1, 1, 3, 5, 5, Pentamethyltrisiloxane (Figure 1, Structure 1).

100 g $tBuMe_2SiCl$ and 46 g $MeHSiCl_2$ were dissolved in 150 ml isopropylether (IPE) and placed in an addition funnel. 150 g water and 250 ml IPE were charged into a 1L round bottom flask equipped with a mechanical stirrer, reflux condenser and $N_2$ inlet. The chlorosilanes were added dropwise via the addition funnel at room temperature (23°C) over a period of 1 h. After addition was completed, the temperature was adjusted to 70°C and the reaction was run at reflux temperature for 20 h and progress followed by GC (88% yield at 20 h). When the reaction was finished, the water was drained off via a separation funnel. The fluid was washed 3 times using 100 g of water each time. 25 g of $NaHCO_3$ was mixed with 100g of water and added slowly to the mixture and stirred for 30 min. The water was again drained and dried over sodium sulfate. After filtering, the IPE was stripped off on the rotor evaporator and the crude product was further fractional distilled under reduced pressure to afford 63g $tBuMe_2SiOMe(H)SiOSi\ Me_2tBu$ (GC purity 97%).

**Figure 1-Rection sequence for preparation of hydride intermediate**

$tBuMe_2SiCl$ + MeHSiCl H$_2$O/IPE $tBuMe_2SiOMe(H)SiOSi\ Me_2tBu$

**Structure 1**

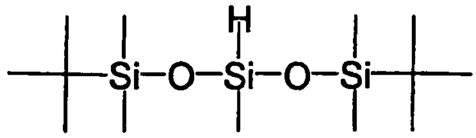

PREPARATION EXAMPLE 2

1,5-di(isopropyl)-1, 1, 3, 5, 5, Pentamethyltrisiloxane (Figure 2, Structure 2).

25 g $iPrMe_2SiCl$ (0.183 moles) and 13.1 g $MeHSiCl_2$ (0.114 moles) were dissolved in 80ml isopropylether (IPE) and placed in an addition funnel. 50g water and 100ml IPE were charged into a 500 ml round bottom flask equipped with a mechanical stirrer, reflux condenser and $N_2$ inlet. The chlorosilanes were added dropwise via the addition funnel at room temperature (23°C) over a period of 40 min. After addition was completed, the temperature was adjusted to 80°C and the reaction was run at reflux temperature for 4 h and progress followed by GC (75% yield at 4 h). When the reaction was finished, the water was drained off via a separation funnel. The fluid was washed 3 times using 80g of water each time. 25 g of $NaHCO_3$ was mixed with 100g of water and added slowly to the mixture and stirred for 30 min. The water was again drained and dried over sodium sulfate. After filtering, the IPE was stripped off on the rotor evaporator and the crude product was further fractional distilled under reduced pressure to afford 10g $iPrMe_2SiOMe(H)SiOSi$ Me2iPr (GC purity 93%).

**Figure 2-Reaction sequence for preparation of hydride intermediate**

$iPrMe_2SiCl$ + MeHSiCl H$_2$O/IPE $iPrMe_2SiOMe(H)SiOSi\ Me_2iPr$

**Structure 2**

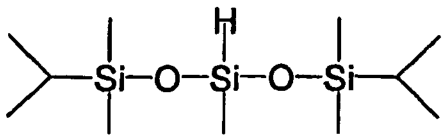

PREPARATION EXAMPLE 3

The hydride intermediates of Examples 1-2 were further modified with various allylpolyalkyleneoxides to yield the organomodified trisiloxane surfactant compositions of the present invention (Table 1), as well as the comparative trisiloxane surfactants (From Table 2).

The organomodified trisiloxane surfactant compositions of the present invention were prepared by conventional methods of platinum mediated hydrosilation, as described in Bailey, U.S. Patent 3,299,112, herein incorporated by reference.

Table 1 provides a description of the compositions of the present invention. Some of these compositions are described by the structure:

M*D'M*

where $M^* = R^1Si(CH_3)_2O_{0.5}$;

D' = $OSi(CH_3)CH_2CH(R^{32})CH_2O-(CH_2CH_2O)_r$ -$(CH_2CH_2O)sR^{33}$ where $R^1$, $R^{32}$, $R^{33}$, subscripts r, and s are described in Table 1.

Table 1-Description of Organomodified Trisiloxane Surfactant Compositions

| I.D. | $R^1$ | $R^{13}$ | r | s | $R^{33}$ |
|---|---|---|---|---|---|
| 1 | $(CH_3)_3C$- | H | 0 | 11 | H |
| 2 | $(CH_3)_2CH$- | H | 0 | 11 | H |

Table 2 provides a description of the comparative trisiloxane and organosilicone polyether based surfactants of the general structure:

$$MD_X D"_Y M$$

where M = $(CH_3)_3SiO_{0.5}$: D = O $Si(CH_3)_2$; and
D" = $OSi(CH_3)CH_2CH_2CH_2O-(CH_2CH_2O)_dR^9$

Table 2- Composition of Comparative Organosilicone Polyether Surfactants

| | | | Polyether Group | |
|---|---|---|---|---|
| I.D. | X | Y | d | $R^9$ |
| A | 0 | 1 | 7.5 | $CH_3$ |
| B | 0 | 1 | 7.5 | H |
| C | 20 | 3 | 7.5 | $CH_3$ |

Additionally, comparative sample OPE (Octylphenolethoxylate, containing 10 polyoxyethylene units) is a non-silicone organic surfactant. This product is available as Triton® X-100 from Dow Chemical Company, Midland, MI.

EXAMPLE 4

This example demonstrates the ability of the organomodified trisiloxane composition of the present invention to reduce aqueous surface tension thereby showing utility as surfactants. Surface tension was measured using a Kruss surface tensiometer, with a sand blasted platinum blade as the sensor. Solutions of the various components were prepared at 0.1 wt% in 0.005M NaCl water (Deionized), as an equilibrium aid.

Table 3 shows that solutions of these unique compositions provide a significant reduction in surface tension relative to the conventional surfactant.

The compositions of the present invention also provide spreading properties similar to the comparative trisiloxane surfactants (A, B). Additionally, organomodified trisiloxane surfactants of the present invention provide improved spreading relative to the conventional silicone polyether (C) and conventional organic surfactant product OPE.

Spreading was determined by applying a 10 μL droplet, of surfactant solution to polyacetate film (USI, "Crystal Clear Write on Film") and measuring the spread diameter (mm) after 30 seconds, at a relative humidity between 50 and 70% (at 22 to 25°C). The solution was applied with an automatic pipette to provide droplets of reproducible volume. Deionized water that was further purified with a Millipore filtration system was used to prepare the surfactant solutions.

Table 3-Surface Tension and Spreading Properties

| | Surface Tension | Spread Diameter (mm) Weight % Surfactant | | | |
|---|---|---|---|---|---|
| I.D. | mN/m | 0.05% | 0.1% | 0.2% | 0.4% |
| 1 | 23.1 | 9 | 11 | 12 | 15 |
| 2 | 23.6 | 10 | 13 | 16 | 25 |
| A | 20.9 | 34 | 53 | 51 | 25 |
| B | 20.6 | 37 | 53 | 50 | 35 |
| C | 23.6 | nd | nd | nd | 6 |

(continued)

| | Surface Tension | Spread Diameter (mm) Weight % Surfactant | | | |
|---|---|---|---|---|---|
| OPE | 31.8 | nd | 9 | nd | 10 |

EXAMPLE 5

Hydrolytic stability was determined for representative compositions of the present invention using HPLC. Solutions of the various compositions were prepared at 0.5 wt% over a pH range from pH 4 to pH 11, and monitored by HPLC for decomposition as a function of time.

Analytical Method

The samples were analyzed by a reverse-phase chromatographic technique using the experimental conditions listed in Table 4.

Table 4-Solvent Gradient for HPLC Method

| Time (min.) | %Methanol | % Water | % Isopropanol |
|---|---|---|---|
| 0.0 | 70 | 30 | 0 |
| 15.0 | 100 | 0 | 0 |
| 20.0 | 50 | 0 | 50 |
| 20.1 | 70 | 30 | 0 |
| 25.0 | 70 | 30 | 0 |

Detector: ELSD/LTA (Evaporative Light Scattering with Low Temperature Adapter

Conditions: 30°C, 1.95 SLPM $N_2$

Column: Phenomenex LUNA C18 end cap, 5 micron, 75x4.6 mm

Flow Rate: 1.0 mL/min.

Inj. Volume: 10 microlitres

Sample: 0.050 g/mL in methanol

Tables 5-8 demonstrates that the compositions of the present invention provide improved resistance to hydrolytic decomposition relative to the standard comparative siloxane based surfactant Siloxane A, under similar pH conditions.

Comparative siloxane A shows rapid hydrolysis at pH values below 5 and at pH values above 7, while the organomodified trisiloxane surfactants of the present invention demonstrates a higher resistance to hydrolysis under the same conditions.

Table 5-Hydrolytic Stability of Siloxane Based Surfactants by HPLC

| | | Stability: % Siloxane Surfactant Remaining | | | | | |
|---|---|---|---|---|---|---|---|
| I.D. | Time | pH 4 | pH 5 | pH 7 | pH 9 | pH 10 | pH 11 |
| 1 | 24h | 100 | 100 | 100 | 100 | 100 | 100 |
| | 1 wk | 100 | 100 | 100 | 100 | 100 | 100 |
| | 2 wk | 100 | 100 | 100 | 100 | 100 | 100 |
| | 4 wk | 100 | 100 | 100 | 100 | 100 | 100 |
| | 6 wk | 100 | 100 | 100 | 100 | 100 | 100 |
| | 9 wk | 100 | 100 | 100 | 100 | 100 | 100 |
| | 12 wk | 100 | 100 | 100 | 100 | 100 | 100 |

(continued)

| | | Stability: % Siloxane Surfactant Remaining | | | | | |
|---|---|---|---|---|---|---|---|
| I.D. | Time | pH 4 | pH 5 | pH 7 | pH 9 | pH 10 | pH 11 |
| | 17 wk | 60 | 100 | 100 | 100 | 100 | 100 |
| | 23 wk | 45 | 100 | 100 | 100 | 100 | 100 |
| | 28 wk | 30 | 100 | 100 | 100 | 100 | 21 |

Table 6-Hydrolytic Stability of Siloxane Based Surfactants by HPLC

| | | Stability % Siloxane Surfactant Remaining | | | | | |
|---|---|---|---|---|---|---|---|
| I.D. | Time | pH 4 | pH 5 | pH 7 | pH 9 | pH 10 | pH 11 |
| 2 | 24 h | 100 | 100 | 100 | 100 | 100 | 100 |
| | 10 days | 100 | 100 | 100 | 100 | 100 | 100 |
| | 5 wk | 100 | 100 | 100 | 100 | 100 | 100 |

Table 8-Hydrolytic Stability of Comparative Siloxane Based Surfactants by HPLC

| | | Stability: % Siloxane Surfactant Remaining | | | | | |
|---|---|---|---|---|---|---|---|
| I.D. | Time | pH4 | pH 5 | pH 7 | pH 9 | pH 10 | pH 11 |
| A | 48h | 25 | 100 | 100 | 100 | 46 | nd |
| | 1 wk | 0 | 38 | 100 | 53 | 0 | nd |

EXAMPLE 7

The impact of other ingredients on spreading was determined by blending the organosilicone disiloxane surfactant of the present invention, with a conventional organic based co-surfactant. The co-surfactants are described in Table 10.

Blends were prepared as physical mixtures where the weight fraction silicone is represented by $\alpha$ (alpha), indicating that the co-surfactant makes up the balance of the blend ratio. For example when $\alpha = 0$ this indicates that the composition contains 0% of the silicone component and 100% co-surfactant, while an $\alpha = 1.0$ indicates the composition contains 100% silicone, and no (0%) co-surfactant. Mixtures of the two components are represented by the weight fraction $\alpha$, where $\alpha$ ranges as follows: $0 \leq \alpha \leq 1.0$. By example when $\alpha = 0.25$ this indicates the surfactant mixture is composed of 25 % silicone and 75 % co-surfactant. These blends are then diluted in water to the desired concentration for spreading evaluation.

Spreading was determined as described in Example 4, at 0.2wt% total surfactant.

Table 11 demonstrates that representative examples of the co-surfactants of the present invention provide favorable spreading results, and in some cases provide an unexpected synergistic enhancement, where the spread diameter of the mixture exceeds that of the individual components.

Table 10 - Description of Conventional Co-surfactants

| ID | Description |
|---|---|
| IDA-5 | Isodecyl alcohol ethoxylate (4-5 EO) |
| IDA-6 | Isodecyl alcohol ethoxylate (5-6 EO) |
| TMN-6 | Trimethylnonylalcohol ethoxylate (6 EO) |
| Oxo-TDA-5 | Oxo-tridecyl alcohol ethoxylate (5 EO) |
| Oxo-TDA-6 | Oxo-tridecyl alcohol ethoxylate (6 EO) |
| APG | $C_{8-10}$ Alkylpolyglucoside |

Table 11- Effect of Co-surfactants on Blend Spreading Properties

| | | Wt Fraction (α) Silicone Surfactant Spread diameter (mm) | | | | | |
|---|---|---|---|---|---|---|---|
| Run | Silicone | 0 | 0.25 | 0.50 | 0.75 | 1.0 | Co-surfactant |
| 1 | 3 | 47 | 24 | 49 | 52 | 55 | IDA-5 |
| 2 | 3 | 33 | 43 | 51 | 53 | 55 | IDA-6 |
| 3 | 3 | 49 | 48 | 54 | 59 | 55 | TMN-6 |
| 4 | 3 | 47 | 37 | 43 | 47 | 55 | Oxo-TDA-5 |
| 5 | 3 | 43 | 34 | 46 | 48 | 55 | Oxo-TDA-6 |
| 6 | 3 | 8 | 50 | 58 | 49 | 55 | APG |

## Claims

**1.** An agricultural composition comprising:

a) a silicone having the formula:

$$M^1D^1M^2$$

wherein

$M^1 = (R^1)(R^2)(R^3)SiO_{1/2}$;
$M^2 = (R^4)(R^5)(R^6)\ SiO_{1/2}$; and
$D^1 = (R^7)(Z)\ SiO_{2/2}$

where

$R^1$ is selected from the group of monovalent hydrocarbon radicals consisting of branched or linear hydrocarbon group consisting of 2 to 4 carbons, aryl, and an alkyl hydrocarbon group of 4 to 9 carbons containing aryl substituents of 6 to 20 carbon atoms; $R^2$, $R^3$ $R^4$, $R^5$, $R^6$ and $R^7$ are each independently selected from the group consisting of 1 to 4 carbon monovalent hydrocarbon radicals, aryl, and a hydrocarbon group of 4 to 9 carbons containing an aryl group; Z is an alkyleneoxide group of the general formula: $R^8(C_2H_4O)_a(C_3H_6O)_b(C_4H_8O)_cR^9$ , where $R^8$ is a linear or branched divalent hydrocarbon radical of 2, 3, 5, 6, 7, 8, or 9 carbon atoms ; $R^9$ is selected from the group consisting of H, monovalent hydrocarbon radicals of from 1 to 6 carbon atoms and acetyl, and the subscripts a, b and c are zero or positive and satisfy the following relationships:

$$2 \leq a + b + c \leq 20 \text{ with } a \geq 2;$$

and

b) an agriculturally active component
wherein said agricultural composition has an enhanced resistance to hydrolysis.

**2.** The composition of claim 1 where $R^1$ and $R^4$ are selected from the group consisting of propyl, iso-propyl, butyl, sec-butyl, iso-butyl and tert-butyl.

**3.** The composition of claim 1 where $R^2$, $R^3$, $R^5$, $R^6$ and $R^7$ are methyl.

**4.** The composition of claim 1 where $R^8$ is a divalent hydrocarbon radical having three carbon atoms.

**5.** The composition of claim 1 where the subscript a ranges from 8 to 12.

**6.** The composition of claim 1 where the subscript b ranges from 0 to 3.

**7.** The composition of claim 1 where the subscript c is 0.

**8.** The composition of claim 1 where $R^9$ is selected from the group consisting of hydrogen and methyl.

**9.** The composition of any of the preceding claims,
wherein said agriculturally active component is selected from the group consisting of
aldimorph, tridemorph, dodemorph, dimethomorph; flusilazol, azaconazole, cyproconazole, epoxiconazole, furconazole, propiconazole, tebuconazole, imazalil, thiophanate, benomyl carbendazim, chlorothialonil, dicloran, trifloxystrobin, fluoxystrobin, dimoxystrobin, azoxystrobin, furcaranil, prochloraz, flusulfamide, famoxadone, captan, maneb, mancozeb, dodicin, dodine, bacillus thuringiensis, spinosad, abamectin, doramectin, lepimectin, pyrethrins, carbaryl, primicarb, aldicarb, methomyl, amitraz, boric acid, chlordimeform, novaluron, bistrifluron, triflumuron, diflubenzuron, imidacloprid, diazinon, acephate, endosulfan, kelevan, dimethoate, azinphos-ethyl, azinphosmethyl, izoxathion, chlorpyrifos, clofentezine, lambda-cyhalothrin, permethrin, bifenthrin, cypermethrin; phenoxy acetic acids, phenoxy propionic acids, phenoxy butyric acids, benzoic acids, triazines and s-triazines, substituted ureas, uracils, bentazon, desmedipham, methazole, phenmedipham, pyridate, amitrole, ctomazone, fluridone, norflurazone, dinitroanilines, isopropalin, oryzalin, pendimethalin, prodiamine, trifluralin, glyphosate, glufosinate, sulfonylureas, imidazolinones, pyridinecarboxylic acids clethodim, diclofop-methyl, fenoxapropethyl, fluazifop-p-butyl, haloxyfop-methyl, quizalofop, sethoxydim, dichlobenil, isoxaben, bipyridylium compounds, zinc sulfate, ferrous sulfate, ammonium sulfate, urea, urea ammonium nitrogen, ammonium thiosulfate, potassium sulfate, monoammonium phosphate, urea phosphate, calcium nitrate, boric acid, potassium salts of boric acid, sodium salts of boric acid, phosphoric acid, magnesium hydroxide, manganese carbonate, calcium polysulfide, copper sulfate, manganese sulfate, iron sulfate, calcium sulfate, sodium molybdate, and calcium chloride.

## Patentansprüche

**1.** Landwirtschaftliche Zusammensetzung, welche umfasst:

a) ein Silikon mit der Formel:

$$M'D'M_2$$

wobei
$M^1 = (R^1)(R^2)(R^3)SiO_{1/2}$;
$M^2 = (R^4)(R)(R^6)\ SiO_{1/2}$; und
$D^1 = (R^7)(Z)\ SiO_{2/2}$
wobei
$R^1$ gewählt ist aus der Gruppe von monovalenten Kohlenwasserstoffradikalen, die aus einer verzweigen oder linearen Kohlenwasserstoffgruppe, die aus 2 bis 4 Kohlenstoffen besteht, Aryl und einer Alkyl-Kohlenwasserstoffgruppe von 4 bis 9 Kohlenstoffen, die Arylsubstituenten von 6 bis 20 Kohlenstoffatomen enthält, bestehen; $R^2$, $R^3$, $R^4$, $R^5$, $R^6$ und $R^7$ jeweils unabhängig aus der Gruppe gewählt sind, die aus monovalenten Kohlenwasserstoffradikalen von 1 bis 4 Kohlenstoffen, Aryl und einer Kohlenwasserstoffgruppe von 4 bis 9 Kohlenstoffen, die eine Arylgruppe enthält, besteht; Z eine Alkylenoxidgruppe der allgemeinen Formel:
$R^8(C_2H_4O)_a(C_3H_6O)_b(C_4H_8O)_cR^9$ ist, wobei $R^8$ ein lineares oder verzweigtes bivalentes Kohlenwasserstoffradikal von 2, 3, 5, 6, 7, 8 oder 9 Kohlenstoffatomen ist; $R^9$ aus der Gruppe bestehend aus H, monovalenten Kohlenwasserstoffradikalen von 1 bis 6 Kohlenstoffatomen und Acetyl gewählt ist und die Tieferstellungen a, b und c null oder positiv sind und die folgenden Beziehungen erfüllen:

$$2 \leq a + b + c \leq 20, \text{ wobei } a \geq 2;$$

und
b) eine landwirtschaftlich aktive Komponente
wobei die landwirtschaftliche Zusammensetzung eine verbesserte Beständigkeit gegenüber Hydrolyse aufweist.

**2.** Zusammensetzung nach Anspruch 1, wobei $R^1$ und $R^4$ aus der Gruppe bestehend aus Poropyl, Isopropyl, Butyl, sec-Butyl, Isobutyl und tert-Butyl gewählt sind.

**3.** Zusammensetzung nach Anspruch 1, wobei $R^2$, $R^3$, $R^5$, $R^6$ und $R^7$ Methyl sind.

**4.** Zusammensetzung nach Anspruch 1, wobei $R^8$ ein bivalentes Kohlenwasserstoffradikal mit drei Kohlenstoffatomen ist.

**5.** Zusammensetzung nach Anspruch 1, wobei die Tieferstellung a von 8 bis 12 reicht.

**6.** Zusammensetzung nach Anspruch 1, wobei die Tieferstellung b von 0 bis 3 reicht.

**7.** Zusammensetzung nach Anspruch 1, wobei die Tieferstellung c 0 ist.

**8.** Zusammensetzung nach Anspruch 1, wobei $R^9$ aus der Gruppe bestehend aus Wasserstoff und Methyl gewählt ist.

**9.** Zusammensetzung nach einem der vorhergehenden Ansprüche,
wobei die landwirtschaftlich aktive Komponente gewählt ist aus der Gruppe bestehend aus
Aldimorph, Tridemorph, Dodemorph, Dimethomorph; Flusilazol, Azaconazol, Cyproconazol, Epoxiconazol, Furconazl, Propiconazol, Tebuconazol, Imazalil, Thiophanat, Benomyl-Carbendazim, Chlorthialonil, Dicloran, Trifloxystrobin, Fluoxystrombin, Dimoxystrobin, Azoxystrobin, Furcaranil, Prochloraz, Flusulfamid, Famoxadon, Captan, Maneb, Mancozeb, Dodicin, Dodin, Bacillus thuringiensis, Spinosad, Abamectin, Doramectin, Lepimectin, Pyrethrins, Carbaryl, Primicarb, Aldicarb, Methomyl, Amitraz, Borsäure, Chlordimeform, Novaluron, Bistrifluron, Triflumuron, Diflubenzuron, Imidacloprid, Diazinon, Acephat, Endosulfan, Kelevan, Dimethoat, Azinphosethyl, Azinphosmethyl, Izoxathion, Chlorpyrifos, Clofentezin, Lambda-Cyhalothrin, Permethrin, Bifenthrin, Cypermethrin; Phenoxyessigsäuren, Phenoxypropionsäuren, Phenoxybuttersäuren, Benzoesäuren, Triazinen und s-Triazinen, substituierten Harnstoffen, Uracilen, Bentazon, Desmedipham, Methazol, Phenmedipham, Pyridat, Amitrol, Clomazon, Fluridon, Norflurazon, Dinitroanilinen, Isopropalin, Oryzalin, Pendimethalin, Prodiamin, Trifluralin, Glyphosat, Glufosinat, Sulfonylharnstoffen, Imidazolinonen, Pyridincarbonsäuren, Clethodim, Diclofopmethyl, Fenoxapropethyl, Fluazifop-p-butyl, Haloxyfopmethyl, Quizalofop, Sethoxydim, Dichlobenil, Isoxaben, Bipyridyliumverbindungen, Zinksulfat, Eisensulfat, Ammoniumsulfat, Harnstoff, Harnstoffammoniumstickstoff, Ammoniumthiosulfat, Kaliumsulfat, Monoammoniumphosphat, Harnstoffphosphat, Calciumnitrat, Borsäure, Kaliumsalzen von Borsäure, Natriumsalzen von Borsäure, Phosphorsäure, Magnesiumhydroxid, Mangancarbonat, Calciumpolysulfid, Kupfersulfat, Mangansulfat, Eisensulfat, Calciumsulfat, Natriummolybdat und Calciumchlorid.

## Revendications

**1.** Composition agricole comprenant:

a) un silicone ayant la formule:

$$M^1 D^1 M^2$$

où
$M^1 = (R^1) (R^2) (R^3) SiO_{1/2}$;
$M^2 = (R^4) (R^5) (R^6) SiO_{1/2}$ ; et
$D^1 = (R^7) (Z) SiO_{2/2}$
où
$R^1$ est sélectionné dans le groupe de radicaux hydrocarburés monovalents consistant en groupes d'hydrocarbures ramifiés ou linéaires consistant en 2 à 4 carbones, aryle, et un groupe d'hydrocarbures d'alkyle de 4 à 9 carbones contenant des substituants d'aryle de 6 à 20 atomes de carbone; $R^2$, $R^3$, $R^4$, $R^5$, $R^6$ et $R^7$ sont chacun indépendamment sélectionnés dans le groupe consistant en 1 à 4 radicaux hydrocarburés monovalents, aryle et un groupe hydrocarbures de 4 à 9 carbones contenant un groupe aryle; Z est un groupe alkylèneoxyde de la formule générale:
$R^8(C_2H_4O)_a(C_3H_6O)_b(C_4H_8O)_cR^9$, où $R^8$ est un radical hydrocarburé divalent linéaire ou ramifié de 2, 3, 5, 6, 7, 8 ou 9 atomes de carbone; $R^9$ est sélectionné dans le groupe consistant en H, radicaux hydrocarburés monovalents de 1 à 6 atomes de carbone et acétyle, et les indices a, b et c sont zéro ou positifs et respondent aux relations suivantes:

$$2 \leq a + b + c \leq 20 \text{ avec } a \geq 2;$$

et
b) un composant actif dans l'agriculture
où ladite composition agricole a une résistance accrue à l'hydrolyse.

**2.** Composition selon la revendication 1, dans laquelle $R^1$ et $R^4$ sont sélectionnés dans le groupe consistant en propyle, iso-propyle, butyle, sec-butyle, iso-butyle et tert-butyle.

**3.** Composition selon la revendication 1, dans laquelle $R^2$, $R^3$, $R^5$, $R^6$ et $R^7$ sont méthyle.

**4.** Composition selon la revendication 1, dans laquelle $R^8$ est un radical hydrocarburé divalent ayant trois atomes de carbone.

**5.** Composition selon la revendication 1, dans laquelle l'indice a est dans une plage de 8 à 12.

**6.** Composition selon la revendication 1, dans laquelle l'indice b est dans une plage de 0 à 3.

**7.** Composition selon la revendication 1, dans laquelle l'indice c est 0.

**8.** Composition selon la revendication 1, dans laquelle $R^9$ est sélectionné dans le groupe consistant en hydrogène et méthyle.

**9.** Composition selon l'une quelconque des revendications précédentes, dans laquelle ledit composant actif dans l'agriculture est sélectionné dans le groupe consistant en aldimorphe, tridémorphe, dodémorphe, diméthomorphe; flusilazole, azaconazole, cyproconazole, époxiconazole, furconazole, propiconazole, tébuconazole, imazalile, thiophanate, bénomyl carbendazime, chlorothialonile, diclorane, trifloxystrobine, fluoxystrobine, dimoxystrobine, azoxystrobine, furcaranile, prochloraze, flusulfamide, famoxadone, captane, manèbe, mancozèbe, dodicine, dodine, bacillus thuringiensis, spinosad, abamectin, doramectin, lepimectin, pyréthrines, carbaryle, primicarbe, aldicarbe, méthomyle, amitraz, acide borique, chlordiméforme, novaluron, bistrifluron, triflumuron, diflubenzuron, imidaclopride, diazinon, acéphate, endosulfane, kélévane, diméthoate, azinphoséthyle, azinphos-méthyle, izoxathion, chlorpyrifos, clofentezine, lambda-cyhalothrine, permethrine, bifenthrine, cypermethrine; phénoxy acides acétiques, phénoxy acides propioniques, phénoxy acides butyriques, acides benzoïques, triazines et s-triazines, urées substituées, uraciles, bentazone, desmedipham, méthazole, phenmédipham, pyridate, amitrole, clomazone, fluridone, norflurazone, dinitroanilines, isopropaline, oryzaline, pendimethaline, prodiamine, trifluraline, glyphosate, glufosinate, sulfonylurées, imidazolinones, cléthodime d'acides pyridinecarboxyliques, diclofop-méthyle, fenoxaprop-éthyle, fluazifop-p-butyle, haloxyfop-méthyle, quizalofop, sethoxydime, dichlobenile, isoxabène, composés de bipyridylium, sulfate de zinc, sulfate ferreux, sulfate d'ammonium, urée, azote ammonium et d'urée, thiosulfate d'ammonium, sulfate de potassium, phosphate de monoammonium, phosphate d'urée, nitrate de calcium, acide borique, sels de potassium de l'acide borique, sels de sodium de l'acide borique, acide phosphorique, hydroxide de magnésium, carbonate de manganèse, polysulfure de calcium, sulfate de cuivre, sulfate de manganèse, sulfate de fer, sulfate de calcium, molybdate de sodium, et chlorure de calcium.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1053678 A
- WO 9422311 A
- WO 2005013693 A
- US 3159601 A
- US 3220972 A
- US 3715334 A
- US 3775452 A
- US 3814730 A, Karstedt
- US 5674832 A
- US 5558806 A
- US 5104647 A
- US 6221811 B
- US 3299112 A, Bailey


### Non-patent literature cited in the description


- Homogeneous Catalysis of Hydrosilation by Transition Metals. **J.L. SPIER.** Advances in Organometallic Chemistry. Academic Press, 2009, vol. 17, 407-447